(19) Europäisches Patentamt
European Patent Office
Office européen des brevets

(11) EP 4 119 585 A1

(12) # EUROPEAN PATENT APPLICATION
published in accordance with Art. 153(4) EPC

(43) Date of publication:
**18.01.2023 Bulletin 2023/03**

(21) Application number: **21767515.6**

(22) Date of filing: **17.02.2021**

(51) International Patent Classification (IPC):
***C08B 37/08** (2006.01)* ***C08J 3/24** (2006.01)*
***A61L 27/20** (2006.01)*

(52) Cooperative Patent Classification (CPC):
**A61L 27/20; C08B 37/003; C08J 3/24**

(86) International application number:
**PCT/KR2021/001989**

(87) International publication number:
**WO 2021/182763 (16.09.2021 Gazette 2021/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.03.2020 KR 20200029805**

(71) Applicant: **Medytox Inc.**
**Chungcheongbuk-do 28126 (KR)**

(72) Inventors:
- **LIM, Cheon Soo**
  **Sejong 30098 (KR)**
- **CHO, Jung Ho**
  **Seoul 06989 (KR)**
- **YANG, Jong Cheol**
  **Jeonju-si Jeollabuk-do 54837 (KR)**
- **RHEE, Chang Hoon**
  **Seoul 04572 (KR)**

(74) Representative: **Brevalex**
**56, Boulevard de l'Embouchure**
**B.P. 27519**
**31075 Toulouse Cedex 2 (FR)**

(54) **CROSSLINKED HYALURONIC ACID AND USE THEREOF**

(57) Provided are a cross-linked hyaluronic acid and use thereof.

FIG. 1

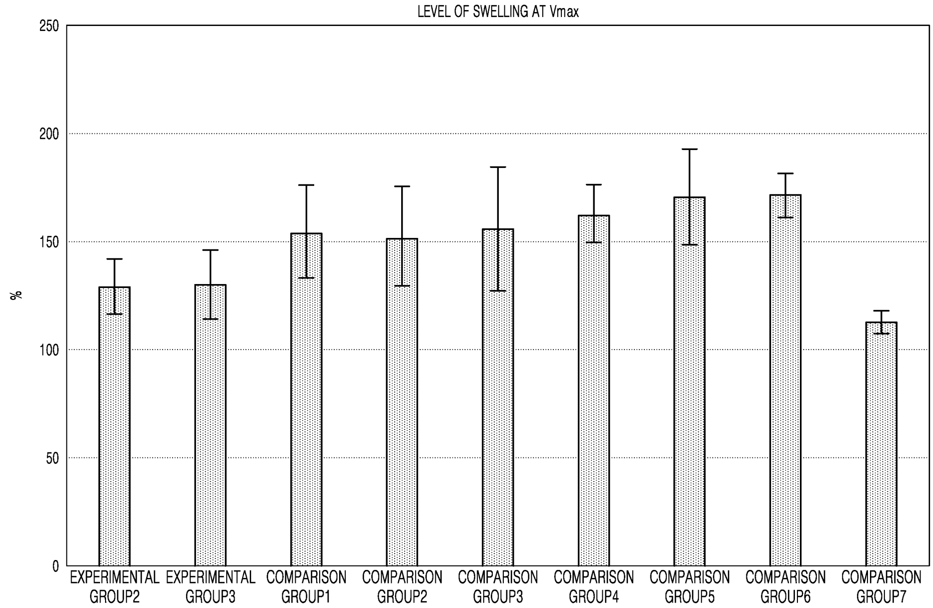


EP 4 119 585 A1

## Description

TECHNICAL FIELD

The present disclosure relates to cross-linked hyaluronic acid and use thereof.

BACKGROUND ART

Hyaluronic acid (HA) is a linear polysaccharide generally having a high average molecular weight. Hyaluronic acid is a polymer of D-glucuronic acid and N-acetyl-D-glucosamine and is negatively charged. Hyaluronic acid is mainly found in extracellular matrix and intercellular matrix, but is also present inside cells. As such, hyaluronic acid is biocompatible because it is a material already present in the body, and also has properties making it easy to prepare as a cross-linked hyaluronic acid hydrogel by using a cross-linking agent or the like. Therefore, filler products using cross-linked hyaluronic acid have been widely used all over the world since Galderma first launched Restylane products in the 1990s.

It is known that a filler product using a cross-linked hyaluronic acid gel may be classified as being monophasic or a biphasic. A monophasic cross-linked hyaluronic acid gel has a high viscous modulus and a low elastic modulus. A biphasic cross-linked hyaluronic acid gel has a low viscous modulus and a high elastic modulus. The reason that monophasic and biphasic cross-linked hyaluronic acid gels show such different properties is because whether the cross-linked hyaluronic acid gel contained in the filler is in a state capable of additionally holding water is different depending on the phase. In the case of a monophasic cross-linked hyaluronic acid gel, since it is in a state capable of holding more water, it is formed as a homogeneous gel with high viscosity and cohesiveness, whereas a biphasic cross-linked hyaluronic acid gel is in a state not capable of holding more water and is formed in the form of hydrogel particles and a solution that is not absorbed into the cross-linked gel rather than as a single homogeneous gel, and thus, the biphasic form has properties of low viscosity and cohesiveness as compared to the monophasic form. Since the biphasic cross-linked hyaluronic acid gel is prepared in the form of particles as described above, it has a high injection force when injected through a syringe needle, and it is known that non-crosslinked hyaluronic acid is added as a lubricant to lower the injection force again.

Due to the different properties of a monophasic cross-linked hyaluronic acid gel and a biphasic cross-linked hyaluronic acid gel, cross-linked hyaluronic acid gels known in the art could have different properties even in the body. For example, in the case of a monophasic cross-linked hyaluronic acid gel with a low elastic modulus and a high viscous modulus, the cohesiveness is excellent, and thus the possibility of breaking away from the injection site is low; however, due to its ability to hold additional water, a monophasic cross-linked hyaluronic acid gel has a property of tending to continuously absorb surrounding water even in the body. For this reason, a monophasic cross-linked hyaluronic acid gel generally has a disadvantage of not retaining the original shape at the initial stage of injection when injected into the body, and increasing volume compared to the previously injected volume. Conversely, a biphasic cross-linked hyaluronic acid gel, in which elastic modulus is high but viscous modulus is low, is able to retain the injected form for a long time with small volume changes, but has properties of easily breaking away from the injection site or not being evenly distributed at the injection site.

WO 2017-213404 A1 provides a cross-linked hyaluronic acid having both monophasic and biphasic properties, and as a method of preparing the same, discloses a production method for a particulated monophasic cross-linked hyaluronic acid. Even according to the related art, there is a demand for a cross-linked hyaluronic acid and uses thereof, wherein the cross-linked hyaluronic acid has a property of high cohesiveness similar to a monophasic cross-linked hyaluronic acid gel, and at the same time, has a property of not being able to hold water similar to a biphasic cross-linked hyaluronic acid gel.

DESCRIPTION OF EMBODIMENTS

TECHNICAL PROBLEM

An aspect provides a cross-linked hyaluronic acid having a critical strain of 0.1 % to 8 % and a tack force of 3 N to 15 N.

Another aspect provides a filler composition including the cross-linked hyaluronic acid.

SOLUTION TO PROBLEM

An aspect provides a cross-linked hyaluronic acid having a critical strain of 0.1 % to 8 % and tack force of 3 N to 15 N.

The term "hyaluronic acid", used herein, refers to hyaluronan or hyaluronate having the formula of Formula 1,

or a pharmaceutically acceptable salt thereof.

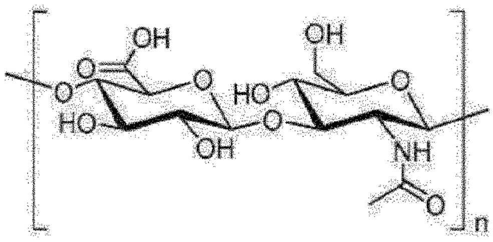

(1)

In Formula 1, n is a number of repeating units. Hyaluronic acid of any origin, including bacterial and algal origin, is useful.

The term "cross-linked", used herein, refers to two or more polymer chains of hyaluronic acid covalently linked via a cross-linking agent. Such cross-linking may be distinguished by intermolecular or intramolecular dehydration that give rise to lactones, anhydrides, or esters within a single polymer or two or more chains. Intramolecular cross-linking may also be included in the composition referred to herein.

The term "cross-linking agent" includes at least two reactive functional groups that generate a covalent bond between two or more molecules. The cross-linking agent may be homobifunctional or heterobifunctional. The cross-linking agent, used herein, may include a functional group complementary to the functional group of hyaluronic acid to allow the cross-linking reaction to proceed.

In the present specification, the term "cross-linked hyaluronic acid" refers to hyaluronic acid which is cross-linked, and "cross-linked hyaluronic acid", or "cross-link hyaluronic acid" are used interchangeably.

An aspect of the present disclosure is to provide a "semiphasic" cross-linked hyaluronic acid that is neither monophasic nor biphasic.

The "semiphasic" cross-linked hyaluronic acid may be defined by a combination of two parameters: critical strain and tack force, and the basis thereof is as described below.

A hydrogel refers to a substance which is a hydrophilic and water-soluble polymer that exists in an insoluble state in water, a solvent, due to reasons such as cross-linking. At this time, the hydrophilic polymer tries to attract water molecules, which are solvents, to the vicinity of the polymer, but since it is not completely dissolved in water, it comes to have various properties. The most notable among the corresponding properties is that a hydrogel is capable of holding a very large amount of water compared to an amount of the polymer. The amount of water that may be held depends on unique properties of a hydrogel such as the type of the polymer, the length of the polymer, and the molecular weight of the polymer, or environment and condition of the solvent to which the hydrogel is exposed, such as the pH of the solvent and the osmotic pressure of the solvent, and when other conditions are the same, the amount of water that may be held depends on how dense the structure of the polymer constituting the hydrogel is. A density of a structure is mainly defined as an average distance between a polymer and another polymer constituting the hydrogel, and the shorter the average distance, the denser the hydrogel may be said. As the hydrogel has a denser structure, the distance between the polymers becomes shorter, and the space for water molecules to exist between the hydrophilic polymers is reduced, which makes it capable of holding a smaller amount of water (Ganji, Fariba & Vasheghani Farahani, Samira & Vasheghani-Farahani, Ebrahim. (2010). Theoretical Description of Hydrogel Swelling: A Review. Iranian Polymer Journal. 19. 375-398). This may be confirmed in a filler prepared by using the hydrogel of cross-linked hyaluronic acid having the corresponding property. In general, fillers currently on sale are cross-linked hyaluronic acid gels diluted to have a concentration of about 20 mg/mL after cross-linking. When cross-linked hyaluronic acid hydrogels having different densities are diluted to have the same concentration, since the amount of moisture that each hydrogel is capable of holding is different, it may be inferred that the appearance after dilution will also be different, and the concept of a phase known in the art may also be expected to originate from the properties of a hydrogel described above. When a cross-linked hyaluronic acid gel is diluted to have a concentration of 20 mg/mL, and there is an additional amount of water that the hydrogel can hold (swelling capacity), the cross-linked hyaluronic acid gel becomes monophasic, and all solutions are absorbed into the cross-linked hyaluronic acid gel and moisture becomes invisible except for the cross-linked gel, and when the amount of moisture exceeds the amount the cross-linked hyaluronic acid gel can hold, a solution coexists with the cross-linked gel, and the phase becomes like a biphase. If so, it may be considered that the monophase, in which only the cross-linked gel exists, and the biphase, in which the cross-linked gel and solution exist together, have different material structures, and the forces acting inside the material structure are also different. What is expected to be a force due to the internal structure of a material may be thought of as the sum of the interactions that make up the internal structure. It consists of a cross-linked hyaluronic acid gel or a cross-linked hyaluronic acid gel and a solution, and interactions that may be confirmed here may be classified into gel-gel interaction, gel-solution interaction, and solution-solution interaction. In case of a solution, the molecular fluidity is higher than that of a gel and the contact area is smaller than that of a gel, so the strength of the interaction may be thought of as gel-gel > gel-solution > solution-solution. Therefore, it may be expected that a monophasic phase, which is a phase in which only the cross-linked gel exists, has a greater internal interaction force than a biphasic phase, which is a phase in which the cross-linked gel and a solution exist together.

Critical strain is a measurement index that identifies the strain value required to cause the destruction of the internal structure of a material, including a linear viscoelastic property, when a shear strain is applied to the material. In case of a monophase in which only the cross-linked hyaluronic acid gel exists without excess solvent, since gel-gel interaction may be considered to be the greatest force constituting the internal force of the material, unlike gel-solution interaction and solution-solution interaction that may be considered to be very small, the monophase has a higher level of interaction and the internal structure may be expected to fail at higher strains. On the other hand, in case of a biphase, the gel-gel interaction is reduced because the solution exists outside the material compared to a monophasic cross-linked hyaluronic acid gel, while the gel-solution interaction and solution-solution interaction become larger, and thus, the total interaction of the internal structure is expected to be smaller compared to that of a monophase, and therefore, the internal structure may be expected to fail at lower strains. Accordingly, the monophasic cross-linked gel capable of holding additional water has a higher critical strain value than the biphasic cross-linked gel that cannot further hold additional water.

In addition, as a result of a similar phenomenon, it may be expected that a typical monophasic filler product has a property of strong cohesiveness, whereas a biphasic filler product has relatively weak cohesiveness compared to a monophasic filler product. The cohesiveness is a property that may be used as a predictive indicator of how well the implant is agglomerated in vivo. To verify this, tack force values, which are indices measuring the degree of cohesiveness, the biphasic filler product and the monophasic filler product, were compared, and as a result, it was confirmed that the monophasic products showed a higher tack force value than the biphasic products.

When in vivo behaviors are predicted with the above descriptions, since a monophasic product has a property of stronger cohesiveness but has a property of being able to additionally hold more water, the monophasic product may become larger than the injected volume when injected into the body, and has a disadvantage of being difficult to control the volume. A biphasic product has an advantage that the volume injected in vivo may be maintained continuously because it cannot hold additional water, but due to low cohesiveness compared to that of a monophasic product, it may be expected have a disadvantage of having possibility of unnatural appearance of skin surface, such as lumps or dents in the injection site, for it is unevenly distributed in the injection site.

It was confirmed that the semiphasic cross-linked gel according to an aspect of the present disclosure have characteristics including all of the characteristics of the existing biphasic and monophasic cross-linked gels. It was confirmed through an in vivo injection test and the low critical strain that a semiphase has a property of not being able to hold more water like the existing biphasic product. At the same time, it was confirmed by a tack force test that it also has a property of high cohesiveness, which is a property of monophasic products in the art. Therefore, in this specification, a semiphasic property is defined as a combination of two parameters: critical strain and tack force.

The cross-linked hyaluronic acid may have a critical strain of 0.1 % to 8.0 %, for example, 0.5 % to 7.0 %, 1.0 % to 7.0 %, or 3.0 % to 7.0 %, and tack force of 3 N to 15 N, for example, 4 N to 15 N, or 4 N to 12 N.

In addition, the cross-linked hyaluronic acid may have an elastic modulus (G') of 400 Pa to 2,000 Pa, for example, 500 Pa to 1,900 Pa, 600 Pa to 1,800 Pa, or 600 Pa to 1,700 Pa.

In addition, the cross-linked hyaluronic acid may have a viscous modulus (G") of 100 Pa to 600 Pa, for example, 120 Pa to 500 Pa, 150 Pa to 450 Pa, or 160 Pa to 350 Pa.

In addition, the cross-linked hyaluronic acid may have a compression of 15 N·s to 100 N·s, for example, 20 N·s to 90 N·s, 25 N·s to 80 N·s, or 30 N • s to 75 N·s.

In addition, the cross-linked hyaluronic acid may have an average injection force of 1 N to 50 N, for example, 3 N to 45 N, 4 N to 40 N, or 7 N to 35 N. The average injection force may be an average injection force when the cross-linked product filled in a 1 mL glass syringe is measured at a speed of 12 mm / min by using a 27 gauge (G), 13 mm long needle.

In addition, the cross-linked hyaluronic acid has an average particle size (μm) of 30 μm to 900 μm, for example, 50 μm to 750 μm, 70 μm to 650 μm, 100 μm to 600 μm, 150 μm to 500 μm, 200 μm to 450 μm, 250 μm to 400 μm, or 250 μm to 350 μm.

In an embodiment, the cross-linked hyaluronic acid has a critical strain of 0.1 % to 8.0 %, and a tack force of 3 N to 15 N, and may be one satisfying one, two, three, four, or five of requirements: i) an elastic modulus (G') of 400 Pa to 2,000 Pa, ii) a viscous modulus (G") of 100 Pa to 600 Pa, iii) compression of 15 N·s to 100 N·s, iv) injection force of 1 N to 50 N, and v) an average particle size (μm) of 30 μm to 900 μm.

In another embodiment, the cross-linked hyaluronic acid has a critical strain of 0.5 % to 7.0 %, and a tack force of 3 N to 15 N, and may be one satisfying one, two, three, four, or five of requirements: i) an elastic modulus (G') of 500 Pa to 1,900 Pa, ii) a viscous modulus (G") of 120 Pa to 500 Pa, iii) compression of 20 N·s to 90 N·s, iv) injection force

of 3 N to 45 N, and v) an average particle size (μm) of 50 μm to 750 μm.

In another embodiment, the cross-linked hyaluronic acid has a critical strain of 1.0 % to 7.0 %, a tack force of 4 N to 15 N, an elastic modulus (G') of 600 Pa to 1,800 Pa, a viscous modulus (G") of 150 Pa to 450 Pa, compression of 25 N·s to 80 N·s, injection force of 5 N to 40 N, and an average particle size (μm) of 70 μm to 650 μm.

In another embodiment, the cross-linked hyaluronic acid has a critical strain of 3.0 % to 7.0 %, a tack force of 4 N to 12 N, an elastic modulus (G') of 600 Pa to 1,700 Pa, a viscous modulus (G") of 160 Pa to 350 Pa, compression of 30 N·s to 75 N·s, injection force of 7 N to 35 N, and an average particle size (μm) of 100 μm to 600 μm.

For the cross-linked hyaluronic acid, before cross-linking, the hyaluronic acid may be derived from any origin. The hyaluronic acid before cross-linking may be, for example, derived from a non-animal organism. The hyaluronic acid before cross-linking may be, for example, derived from bacteria. The bacteria may be from the genus *Streptococcus.* The *Streptococcus* genus bacteria may be *Streptococcus equi, S. pyogenes* or *S. zooepidemicus.* The hyaluronic acid before cross-linking may also be commercially purchased. Intrinsic viscosity of the hyaluronic acid before cross-linking may be 1.0 $m^3$/kg to 4.0 $m^3$/kg.

The cross-linked hyaluronic acid may be prepared by a method including incubating a reaction mixture including a cross-linking agent and hyaluronic acid to proceed a cross-linking reaction. The cross-linking agent may have a multifunctional group. The cross-linking agent may have a difunctional epoxy group. The cross-linking agent having a difunctional epoxy group may be at least one selected from the group consisting of 1,4-butanediol diglycidyl ether, poly(ethylene glycol) diglycidyl ether, poly(propylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, and 1,6-hexanediol diglycidyl ether.

The cross-linked hyaluronic acid may have a cross-link rate of 0.5 % to 7 %. The cross-link rate may be, for example, 0.6 % to 7 %, 0.7 % to 7 %, 0.8 % to 7 %, 0.9 % to 7 %, 1.0 % to 7 %, 1.5 % to 7 %, 2.0 % to 7 %, 2.5 % to 7 %, 3.0 % to 7 %, 1.5 % to 4.5 %, 3.0 % to 4.0 %, 3.2 % to 4.0 %, 3.4 % to 3.8 %, 1.0 % to 4.0 %, or 1.5 % to 4.0 %.

The term "cross-link rate (%)", used herein, refers to a ratio of covalently bonded hyaluronic acid disaccharide repeating units per disaccharide repeating unit of hyaluronic acid. The disaccharide repeating unit consists of D-glucuronic acid and N-acetylglucosamine. The cross-link rate may be identified by a known method. For example, the cross-link rate may be identified by ion exchange chromatography (IEC) or nuclear magnetic resonance spectroscopy (NMR). Ion exchange chromatography is a method of separating and analyzing analytes by using a difference in affinity for the stationary phase by performing reversible ion exchange between a stationary phase and a mobile phase. When using IEC, the formula for calculating the cross-link rate is as follows.

$$\text{Cross-link rate (\%)} = \sum(\text{peak area} \times \text{number of disaccharide repeating units of hyaluronic acid} \times \text{ratio of cross-linked disaccharide repeating units}) \times 100 / \sum(\text{peak area} \times \text{number of disaccharide repeating units of hyaluronic acid})$$

The cross-linked hyaluronic acid may have a semiphasic property that is neither monophasic nor biphasic. The cross-linked hyaluronic acid may be one having a property of high cohesiveness similar to that of a monophasic cross-linked hyaluronic acid, and at the same time, having a property of not being able to hold more water similar to that of a biphasic cross-linked hyaluronic acid.

Another aspect provides a filler composition including the cross-linked hyaluronic acid.

In the composition, the final concentration of the cross-linked hyaluronic acid gel may be 10 mg/ml or more, 15 mg/ml or more, 18 mg/ml or more, or 19 mg/ml or more. For example, the concentration of the cross-linked hyaluronic acid may be 10 mg/ml to 30 mg/ml, 12 mg/ml to 28 mg/ml, 14 mg/ml to 26 mg/ml, 16 mg/ml to 24 mg/ml, 18 mg/ml to 22 mg/ml, or about 20 mg/ml.

The composition may be used for tissue repair in a subject.

For the composition, the subject may be a mammal. The mammal may be a human, a dog, a cat, a cow, a pig, a rat, or sheep.

The composition may be one not additionally including non-crosslinked hyaluronic acid. "Additionally including" non-crosslinked hyaluronic acid means intentionally adding more non-crosslinked hyaluronic acid to a cross-linked hyaluronic acid, and not additionally including non-crosslinked hyaluronic acid means not intentionally adding more non-crosslinked hyaluronic acid to a cross-linked hyaluronic acid.

The composition may further include an anesthetic. The anesthetic may be a local anesthetic. The anesthetic may be at least one selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl paminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof, but is not limited thereto.

The composition may not contain a pharmaceutically active substance selected from the group consisting of proteins, glycosaminoglycans other than hyaluronic acid, and hydroxypropyl methyl cellulose.

The composition may be filled in a syringe.

The composition may be for one or more uses selected from the group consisting of facial plastic surgery, wrinkle improvement, facial contouring, breast plastic surgery, breast augmentation, genital enlargement, glans enlargement, urinary incontinence treatment, and arthritis treatment.

In the composition, the injection force may be 50 N or less when the composition filled in a 1 ml syringe is measured at a speed of 12 mm/min by using a 27 gauge (G), 13 mm needle. The injection force may be, for example, 1 N to 50 N, 3 N to 45 N, 4 N to 40 N, or 5 N to 35 N.

The composition may further include a pharmaceutically acceptable carrier, excipient and diluent. The carrier may include, for example, water or a buffer. The buffer may be such that the pH of the solution changes very little with the addition of the components of the composition. The composition may be an aqueous liquid composition. The composition may be an aqueous buffered composition. The pH of the aqueous buffered composition may be in the physiological pH range, for example, between about 6.0 and 8.0. The pH may be adjusted by adding a suitable acid or base, such as HCl, $Na_2CO_3$ or NaOH. In an embodiment, the aqueous buffered composition may include phosphate buffered saline (PBS). In another embodiment, the aqueous buffered composition may include tris(hydroxymethyl)aminomethane (Tris). In some embodiments, additional solutes, such as sodium chloride, calcium chloride, and potassium chloride, may be added to adjust osmolarity and ionic concentration.

The composition may be one in which the cross-linked hyaluronic acid is suspended in an aqueous solvent. The aqueous solvent may be a buffer such as PBS, water or saline. The composition may be sterile.

The composition may be contained in a container. The container may be a syringe. The composition may be previously filled into a syringe before use. The composition may be administered with a pre-filled syringe.

Another aspect provides a device including the composition described above. The device may be a pre-filled syringe. The device may be sterile.

Another aspect provides a kit including the above-described pre-filled syringe. The kit may include instructions for administering the composition.

Another aspect provides a method of filling a tissue of a subject including administering to the subject a therapeutically effective amount of the above-described composition. The method may be for augmentation, repair or strengthening of a tissue of a subject or for filling a body cavity. From this point of view, volume augmentation may be a long-lasting increase in volume by a component forming the filler composition. The component forming the filler composition may be one that does not undergo rapid diffusion. In the method, "composition" and "subject" are as described above. The administration may be administering into the skin such as the dermis, or joint cavity. In the method, the administration may be administrating through a syringe, for example, a pre-filled syringe, into the skin, for example, into the dermis. In the method, the administration may be administering 0.1 ml to 50 ml, 0.5 ml to 30 ml, 0.5 ml to 20 ml, 0.5 ml to 15 ml, or 0.8 ml to 12 ml per one time. In the method, the administering may be administering the composition once per period of 3 months or more, once per period of 4 months or more, once per period of 5 months or more, once per period of 6 months or more, once per period of 12 months or more, or once per period of 18 months or more.

## ADVANTAGEOUS EFFECTS OF DISCLOSURE

Provided are a cross-linked hyaluronic acid, which has a property of high cohesiveness similar to a monophasic cross-linked hyaluronic acid gel, and at the same time has properties of not being able to hold water similar to a biphasic cross-linked hyaluronic acid gel, and uses thereof.

## BRIEF DESCRIPTION OF DRAWINGS

FIG. 1 is a diagram showing measured maximum volumes of the injection site compared to the initial volume after injecting cross-linked hyaluronic acid into hairless mice.

FIG. 2 is a diagram showing volumes of the injected hyaluronic acid gels according to the time elapsed after cross-linked hyaluronic acid samples are injected into hairless mice.

BEST MODE

Hereinafter, the present disclosure will be described in more detail through embodiments. However, these embodiments are intended to illustrate the present disclosure, and the scope of the present disclosure is not limited to these embodiments.

**Materials and methods**

The following materials and methods were used in the following embodiments.

**(1) Material: Cross-linked hyaluronic acid product**

As control groups, commercially available cross-linked hyaluronic acid products as follows were used. As monophasic hyaluronic acids, Allergan's Juvederm Voluma Lidocaine™, Hanmi Pharmaceutical's Gugufill™, and Medytox's Neuramis Volume Lidocaine™ were purchased. In addition, as a biphasic hyaluronic acid, Galderma's Restylane Lyft Lidocaine™ was purchased.

**(2) Method**

(2.1) Critical strain measurement method

Critical strain was measured by a strain-sweep test. Specifically, a DHR-2 rheometer instrument (TA instruments) was operated. For the strain-sweep test, temperature of the device was set to 25 °C, and a geometry of a diameter of 25 mm was equipped to perform calibration. An appropriate amount of a sample was loaded in the center between an upper Peltier plate and a lower geometry. The sample was overloaded with a sufficient amount so as not to be insufficient, and an appropriate amount was loaded so that it does not stain the side and top surfaces of the geometry, even if the residue outside the lower area of the geometry was to be trimmed. After lowering the geometry to a set distance, and checking whether the sample is well filled under the geometry, the remaining sample outside the geometry was trimmed and removed. Then, when a strain of 0.1% to 1,000 % was applied to the sample by using the geometry, the value of the elastic modulus measured at each strain was measured. A semi-log graph was drawn with the value obtained by taking log of the strain % as the x-axis and the elastic modulus as the y-axis. When a graph differentiated from this graph value was drawn, the lowest strain % value among the strain % values having the minimum y value was defined as the critical strain.

**(2.2) Tack force measurement method**

Tack force was measured as follows. A DHR-2 rheometer instrument (TA instruments) was operated. For the tack force test, temperature of the device was set to 25 °C, and a geometry of a diameter of 40 mm was equipped to perform calibration. An appropriate amount of the sample was loaded in the center between an upper Peltier plate and a lower geometry. The sample was overloaded with a sufficient amount so as not to be insufficient, and an appropriate amount was loaded so that it does not stain the side and top surfaces of the geometry. After lowering the geometry to a set distance, and checking whether the sample is well filled under the geometry, the remaining sample outside the geometry was trimmed and removed. Thereafter, the force applied to the geometry was measured when the geometry gap was set to a height of 1,000 μm and the geometry was pulled from the lower Peltier plate at a constant speed of 100.0 μm/s in the vertical axis direction for 180 seconds. Due to the tack force of the sample between the geometry and the Peltier plate, the greatest force is measured at the moment the initial sample is separated, and the corresponding force was defined as the tack force.

**(2.3) Viscoelasticity measurement method**

A DHR-2 rheometer instrument (TA instruments) was operated. For the viscoelasticity test, temperature of the device was set to 25 °C, and a geometry of a diameter of 40 mm was equipped to perform calibration. An appropriate amount of the sample was loaded in the center between an upper Peltier plate and a lower geometry. The sample was overloaded with a sufficient amount so as not to be insufficient, and an appropriate amount was loaded so that it does not stain the side and top surfaces of the geometry. After lowering the geometry to a set distance, and checking whether the sample is filled under the geometry, the remaining sample outside the geometry was trimmed and removed. Then, a shear strain was periodically applied to the sample according to a specific frequency with a constant strain of the geometry, and among the modulus values obtained, the storage modulus and loss modulus values at a frequency of

0.1 Hz were defined as elasticity and viscosity, respectively.

**(2.2) Injection force measurement method**

Injection force was measured by using Mecmesin's Multitest 2.5-i Universal Testing Machine. As a load cell for measuring the force applied to the device, an appropriate load cell with a higher allowable value than the measurement range of injection force was used. A syringe containing the sample was placed under the load cell of the instrument, and a 27G 1/2" needle was attached to the syringe. A flat-tipped push bar was placed with the syringe so that a constant force was applied when the load cell applied force. After adjusting the distance so that the load cell part was placed just before it would touch the tip of the push bar, the force applied was measured by pressing the push bar by using the load cell at a speed of 12 mm/min. In case of the initial part and the end part of the measured value, the pressure value generated when the sample flows into the needle was lower than the pressure applied when the syringe was injected. In addition, when the injection of the infusion is almost completed, there may be a case in which a force is continuously applied even when there is no sample, and in this case, the applied force is a force not related to the injection force of the sample. Therefore, the values corresponding to parts where the sample flowed into the needle, and where the injection was almost completed were excluded, and the average value of the injection force measured in the middle part of the syringe filling solution was defined as the injection force, excluding the measured values of the initial injection force and end injection force.

**(2.2) Compression measurement method**

A DHR-2 rheometer instrument (TA instruments) was operated. For the compression test, temperature of the device was set to 25 °C, and a geometry of a diameter of 25 mm was equipped to perform calibration. 1 mL of the sample was loaded in the center between an upper Peltier plate and a lower geometry. After the geometry was lowered to a set distance, the geometry was rotated at a slow speed so that the sample could come to the center with respect to the geometry. Thereafter, while lowering the geometry from 2,500 μm to 900 μm at a constant speed of 13.33 μm/s, the force applied to the geometry was measured. When a graph was drawn with the force measured from the beginning to the end of the test as the Y axis and the movement time as the X axis, compression was defined as a value corresponding to the value of the area under the graph, which is an integral value of the graph.

**(2.6) Particle size measurement method**

Particle size measurement was performed by using Microtrac's Particle Size Analyzer S3500. Particle size measurement was performed by adding a sample to a solvent, and was performed by using laser diffraction analysis method. The solvent was measured using distilled water. After thoroughly washing the sample inlet with distilled water before performing particle size measurement, 1.33 and 1.37 were input as refractive index of the distilled water and the refractive index of the sample, respectively, and the sample type was set to be transparent and irregularly shaped particles. Before measurement, after filling the sample inlet of the device with distilled water, the sample was put into a tube, etc., and diluted with excess distilled water, and then was sufficiently dispersed by using a Vortex, etc., and then the sample was put into the device for measurement. Among the measurement results, the average particle size (D50) was used as data.

**Example: Preparation of cross-linked hyaluronic acid and identification of physical properties**

1. Preparation of cross-linked hyaluronic acid

A cross-linked hyaluronic acid having a critical strain of 0.1 % to 8 % and a tack force of 3 N to 15 N (hereinafter referred to as "experimental group") was prepared as follows.

**Experimental Group 1:**

First, a 1 % (w/w) NaOH solution was prepared. 5 g of sodium hyaluronate (IV 1.7 to 1.9) was mixed with the prepared 1 % NaOH solution to 14.50 % (w/w), and then stirred to sufficiently dissolve. Here, IV represents intrinsic viscosity. 0.528 g of butanediol diglycidyl ether (BDDE) (Sigma-Aldrich) was added to this solution and further stirred to mix well, and then the solution was taken out and a cross-linking reaction was proceeded at 25 °C for 18 hours to prepare a cross-linked hyaluronic acid gel. Next, the obtained cross-linked gel was placed in a dialysis membrane and sealed, and dialysis was performed using 1 mol/kg NaCl aqueous solution and 1x PBS aqueous solution as dialysis solutions. After dialysis is completed, the weight of sodium hyaluronate, in which cross-linking and dialysis has been completed,

was calculated by considering the loss rate, a content calibration was proceeded using 1x PBS so that the concentration of the total sodium hyaluronate would be 2 % (w/w), at this time, the content calibration was proceeded so that a content of lidocaine hydrochloride hydrate would be 0.3 % (w/w). As a result, a PBS solution containing 2 % (w/w) of cross-linked hyaluronic acid and 0.3 % of lidocaine was prepared. After filling 1 ml of the prepared cross-linked hyaluronic acid-containing solution into a glass syringe, high-temperature steam sterilization was performed.

**Experimental Group 2:**

First, a 1 % (w/w) NaOH solution was prepared. 5 g of sodium hyaluronate (IV 1.7 to 1.9) was mixed with the prepared 1 % NaOH solution to 14.0 % (w/w), and then stirred to dissolve for 6 hours. 0.428 g of BDDE (Sigma-Aldrich) was added to this solution, and the mixture was further stirred for 10 minutes to mix well, and then the solution was taken out and left at 25 °C for 18 hours to proceed a cross-linking reaction to prepare a cross-linked hyaluronic acid gel. Next, after dialysis and content calibration were performed in the same manner as with Experimental Group 1, a glass syringe was filled.

**Experimental Group 3:**

First, a 1 % (w/w) NaOH solution was prepared. 5 g of sodium hyaluronate (IV 1.7 to 1.9) was mixed with the prepared 1 % NaOH solution to 15.0 % (w/w), and then stirred to dissolve for 6 hours. 0.251 g of BDDE (Sigma-Aldrich) was added to this solution, and the mixture was further stirred for 10 minutes to mix well, and then the solution was taken out and left at 40 °C for 18 hours to proceed a cross-linking reaction to prepare a cross-linked hyaluronic acid gel. Next, after dialysis and content calibration were performed in the same manner as with Experimental Group 1, a glass syringe was filled.

**Comparison Groups 1 to 3**

Cross-linked hyaluronic acids of Comparison Groups 1 to 3 were prepared in the same manner as with Experimental Group 1, except that the hyaluronic acid dissolution concentration, amount of added BDDE, cross-linking temperature, and cross-linking time were as described in Table 1 below. Table 1 shows the materials and conditions used for preparing the cross-linked hyaluronic acids of Experimental Groups 1 to 3 and Comparison Groups 1 to 3. In case of the BDDE concentration, it represents % of the number of moles of the added BDDE to the number of moles of the added hyaluronic acid (HA) monomers.

[Table 1]

| Name | HA (%, w/w) | BDDE (mol%) | Temperature (°C) | Hour (h) |
|---|---|---|---|---|
| Experimental Group 1 | 14.5 | 21 | 25 | 18 |
| Experimental Group 2 | 14.0 | 17 | 25 | 18 |
| Experimental Group 3 | 15.0 | 10 | 40 | 18 |
| Comparison Group 1 | 12.0 | 22 | 25 | 18 |
| Comparison Group 2 | 11.0 | 21 | 25 | 18 |
| Comparison Group 3 | 11.0 | 16 | 25 | 18 |

Sensory evaluation was performed by using a sense of touch on the samples prepared in the experimental groups and the comparison groups. That is, the phases of the prepared material were identified with a sense of touch, and it was confirmed that, in case of a monophase, the particles were not palpable, and in case of a biphase, the particles were touched like grains of sand. In the sensory evaluation, the cross-linked hyaluronic acid gels of Experimental Groups 1 to 3 had a gel shape that was neither monophasic nor biphasic. On the other hand, the cross-linked hyaluronic acid gels of Comparison Groups 1 to 3 were confirmed through the sensory evaluation as having a monophase.

**Comparison Groups 4 to 7:**

As Comparison Groups 4 to 6, Allergan's Juvederm Voluma Lidocaine™ (Comparison Group 4), Hanmi Pharmaceutical's Gugufill™ (Comparison Group 5), and Medytox's Neuramis Volume Lidocaine™ (Comparison Group 6), which are monophasic cross-linked hyaluronic acids, were respectively used. In addition, as Comparison Group 7,

Galderma's Restylane Lyft Lidocaine™ (Comparison Group 7), which is a biphasic cross-linked hyaluronic acid, was used.

## 2. Analysis of physical properties of prepared cross-linked hyaluronic acid

The physical properties of the cross-linked hyaluronic acid gel prepared in Section 1 were analyzed. The measured physical properties of the prepared cross-linked hyaluronic acids of Experimental Groups 1 to 3, and Comparison Groups 1 to 7 are as shown in Table 2.

[Table 2]

| Name | G' (Pa) | G" (Pa) | Particle size (μm) | Degree of cross-linking (%) | Compression (Ns) | Tack force (N) | Injection force (N) | Critical strain (%) |
|---|---|---|---|---|---|---|---|---|
| Experimental Group 1 | 1629 | 450.3 | 358.3 | 3.20 | 65.20 | 7.46 | 16.01 | 3.36 |
| Experimental Group 2 | 1244.0 | 336.1 | 303.4 | 2.62 | 71.74 | 10.65 | 30.28 | 5.81 |
| Experimental Group 3 | 609.4 | 164.3 | 241.6 | 2.73 | 40.68 | 6.73 | 11.95 | 6.98 |
| Comparison Group 1 | 871.8 | 210.0 | 269.3 | 3.38 | 55.38 | 11.72 | 31.90 | 60.69 |
| Comparison Group 2 | 614.5 | 135.1 | 304.9 | 3.47 | 54.72 | 11.02 | 24.19 | 59.34 |
| Comparison Group 3 | 391.6 | 76.7 | 366.2 | 2.74 | 46.00 | 8.45 | 18.57 | 103.02 |
| Comparison Group 4 | 227.4 | 30.3 | 660.7 | 2.08 | 23.79 | 4.50 | 8 (27G) | 54.62 |
| Comparison Group 5 | 389.0 | 57.0 | N/A | 5.00 | 48.71 | 7.07 | 32.59 | 85.27 |
| Comparison Group 6 | 305 | 50 | 410 | 2.75 | 76.30 | 6.94 | 17 | 112.01 |
| Comparison Group 7 | 742.7 | 203.3 | 809.0 | 0.16 | 22.39 | 3.22 | 19 (29G) | 8.19 |

In Table 2, the injection force indicates the injection force when a 13 mm needle of 27 G is used, unless otherwise specified.

## 3. Measurement of lifting and volumizing effects when the prepared cross-linked hyaluronic acid is applied to animals

Cross-linked hyaluronic acids of Experimental Groups 2 and 3 and Comparison Groups 1, 2, 3, 4, 5, 6 and 7 and saline as a negative control group were injected into animals, and the height, maximum length, and volume of the injection site were measured. For animals, a total of 84 hairless 6-week-old female (18.0 g to 24.0 g, average 22.3 g) mice (SKH1-hr) were used. Eight mice were used per sample, and four were used for saline, which is a control group.

Specifically, 0.1 ml of each of the cross-linked hyaluronic acid samples was administered subcutaneously to one dorsal region site per hairless mouse using a glass syringe. After injection, the height, maximμm length and volume of the injection site were measured by using a Primose device (Primos 5.8E (Canfield Scientific Inc, NJ, USA)) according to designated days. In addition, the injection site was visually observed and photographs were taken. As a result of visual observation, Comparison Groups 1, 2, 3, 4, 5, and 6 showed high swelling.

FIG. 1 is a diagram showing measured maximum volumes of the injection site compared to the initial volume after injecting cross-linked hyaluronic acid into hairless mice. As shown in FIG.1, it was confirmed that the volume increase of Experimental Groups 2, 3 and Comparison Group 7 was smaller than that of Comparison Groups 1, 2, 3, 4, and 5. This indicates that the cross-linked hyaluronic acid gels of Experimental Groups 2 and 3 caused little swelling at

the injection site.

FIG. 2 is an observation of the volume change up to 180 days after intradermally injecting cross-linked hyaluronic acid gels. As shown in FIG. 2, in case of monophases, the volume of cross-linked hyaluronic acids of Comparison Groups 2 and 5 increased by 168% and 143 %, respectively, compared to the initial volume at 4 weeks after injection. As shown in FIG. 2, at 180 days after injection, the cross-linked hyaluronic acids of Experimental Groups 2 and 3 were larger or equivalent in volume to other comparison groups and the control group. This indicates that the cross-linked hyaluronic acids of Experimental Groups 2 and 3 showed relatively small loss during 180 days. That is, the cross-linked hyaluronic acids of Experimental Groups 2 and 3 had superior in vivo durability compared to other comparison groups and the control group.

In conclusion, it was confirmed that Experimental Groups 2 and 3 exhibited smaller swelling after injection compared to Comparison Groups 1, 2, 3, 4, 5 and 6, and thus, the volume change after injection was small, and it was confirmed that the volumes of Experimental Groups 2 and 3 up to 180 days after injection were equivalent to that of Comparison Groups 1, 2, 3, 4, 5 and 6, and was superior to that of Comparison Group 7, and thus, in vivo durability was confirmed to be excellent.

### 4. Irritation measurement when prepared cross-linked hyaluronic acid gel is applied to animals: irritation test

Cross-linked hyaluronic acids of Experimental Groups 2 and 3 and Comparison Groups 1, 2, 3, 4, 5, 6 and 7 and saline as a negative control group were injected into animals, and the degree of irritation induced at the injection site was identified by measuring erythema and edema. The experiment was performed according to the guideline described in ISO 109993-10 "Tests for irritation and skin sensitization" by entrusting to Knotus Co., Ltd. (Korea). As animals, a total of 12 male New Zealand white rabbits were selected according to ISO 10993-10. Three rabbits were used per sample. Specifically, 200 μl of each of the cross-linked hyaluronic acid samples per rabbit was subcutaneously administered to 5 dorsal region sites and the injection sites were observed for 45 days. After injection, erythema and edema were evaluated and scored according to the guideline. The differences between the score for each sample and the evaluation score for the control group using saline were cumulatively added and averages for each site were obtained. As a result, no erythema was observed in any animal to which the samples were administered. Table 3 is a table showing the edema scores observed on days 3 and 45 after administering cross-linked hyaluronic acid samples to rabbits. There was no statistically significant difference in the edema scores among the administered groups of animals to which samples were administered.

[Table 3]

| Sample | Edema score at day 3 |
|---|---|
| Experimental Group 2 | 0.6 |
| Experimental Group 3 | 0.9 |
| Comparison Group 1 | 1.3 |
| Comparison Group 2 | 1.3 |
| Comparison Group 3 | 1.8 |
| Comparison Group 4 | 1.1 |
| Comparison Group 5 | 0.8 |
| Comparison Group 6 | 0.9 |
| Comparison Group 7 | 1.1 |
| Saline | 0.0 |

According to the ISO 109993-10 guideline, as a result of measuring formation of erythema and edema up to 3 days, the samples of Experimental Groups 2 and 3 and Comparison Groups 5 and 6 met the criteria of the guideline. Therefore, from the edema formation results on the 3rd day of injection, the samples of Experimental Groups 2 and 3 were confirmed to show equal or superior effects compared to the samples of other comparison groups.

In the above Example, the cross-linked hyaluronic acid gels of Experimental Groups 2 and 3 were confirmed to have superior properties compared to the cross-linked hyaluronic acid gels of other comparison groups in terms of volume increase resistance and low-irritation when injected into animals.

## Claims

1. A cross-linked hyaluronic acid having a critical strain of 0.1 % to 8 % and a tack force of 3 N to 15 N.

2. The cross-linked hyaluronic acid of claim 1, wherein an elastic modulus (G') of the cross-linked hyaluronic acid is 400 Pa to 2,000 Pa.

3. The cross-linked hyaluronic acid of claim 1 or claim 2, wherein a viscous modulus (G") of the cross-linked hyaluronic acid is 100 Pa to 600 Pa.

4. The cross-linked hyaluronic acid of any one of claims 1 to 3, wherein compression of the cross-linked hyaluronic acid is 15 N·s to 100 N·s.

5. The cross-linked hyaluronic acid of any one of claims 1 to 4, wherein an injection force of the cross-linked hyaluronic acid is 1 N to 50 N.

6. The cross-linked hyaluronic acid of any one of claims 1 to 5, wherein the cross-linked hyaluronic acid is cross-linked by a cross-linking agent having a difunctional epoxy group.

7. The cross-linked hyaluronic acid of claim 6, wherein the cross-linking agent having a difunctional epoxy group is at least one selected from the group consisting of 1,4-butanediol diglycidyl ether, poly(ethylene glycol) diglycidyl ether, poly(propylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, and 1,6-hexanediol diglycidyl ether.

8. A filler composition comprising the cross-linked hyaluronic acid of any one of claims 1 to 7.

9. The filler composition of claim 8, wherein a concentration of the cross-linked hyaluronic acid is 10 mg/mL to 30 mg/mL.

10. The filler composition of claim 8 or claim 9, not comprising additional non-crosslinked hyaluronic acid.

11. The filler composition of any one of claims 8 to 10, further comprising a local anesthetic.

12. The filler composition of any one of claims 8 to 11, wherein the filler composition is filled in a syringe.

13. The filler composition of any one of claims 8 to 12, wherein the composition is for one or more uses selected from the group consisting of facial plastic surgery, wrinkle improvement, facial contouring, breast plastic surgery, breast augmentation, genital enlargement, glans enlargement, urinary incontinence treatment, and arthritis treatment.

FIG. 1

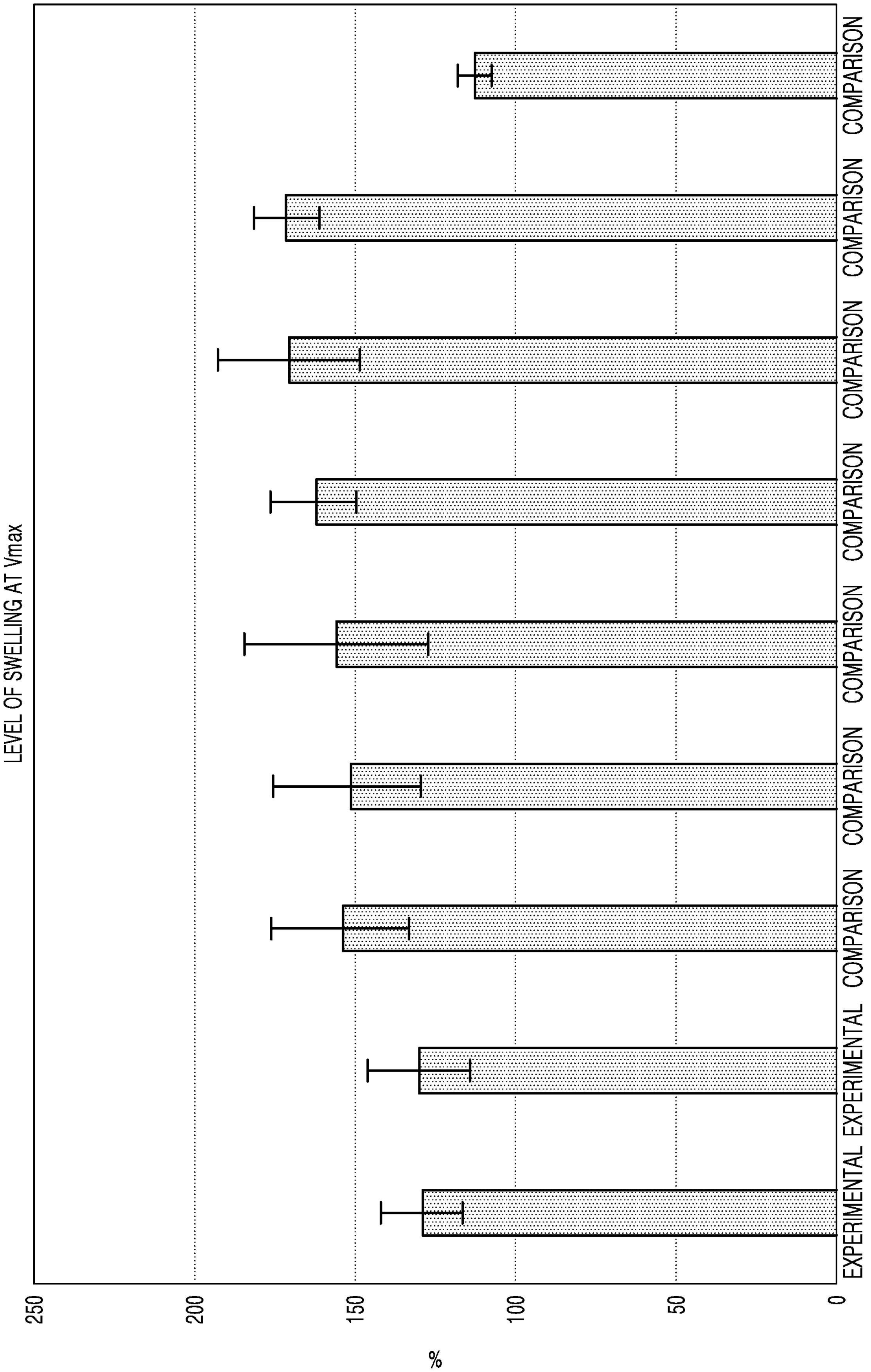
LEVEL OF SWELLING AT Vmax
%
250
200
150
100
50
0
EXPERIMENTAL GROUP2
EXPERIMENTAL GROUP3
COMPARISON GROUP1
COMPARISON GROUP2
COMPARISON GROUP3
COMPARISON GROUP4
COMPARISON GROUP5
COMPARISON GROUP6
COMPARISON GROUP7

FIG. 2

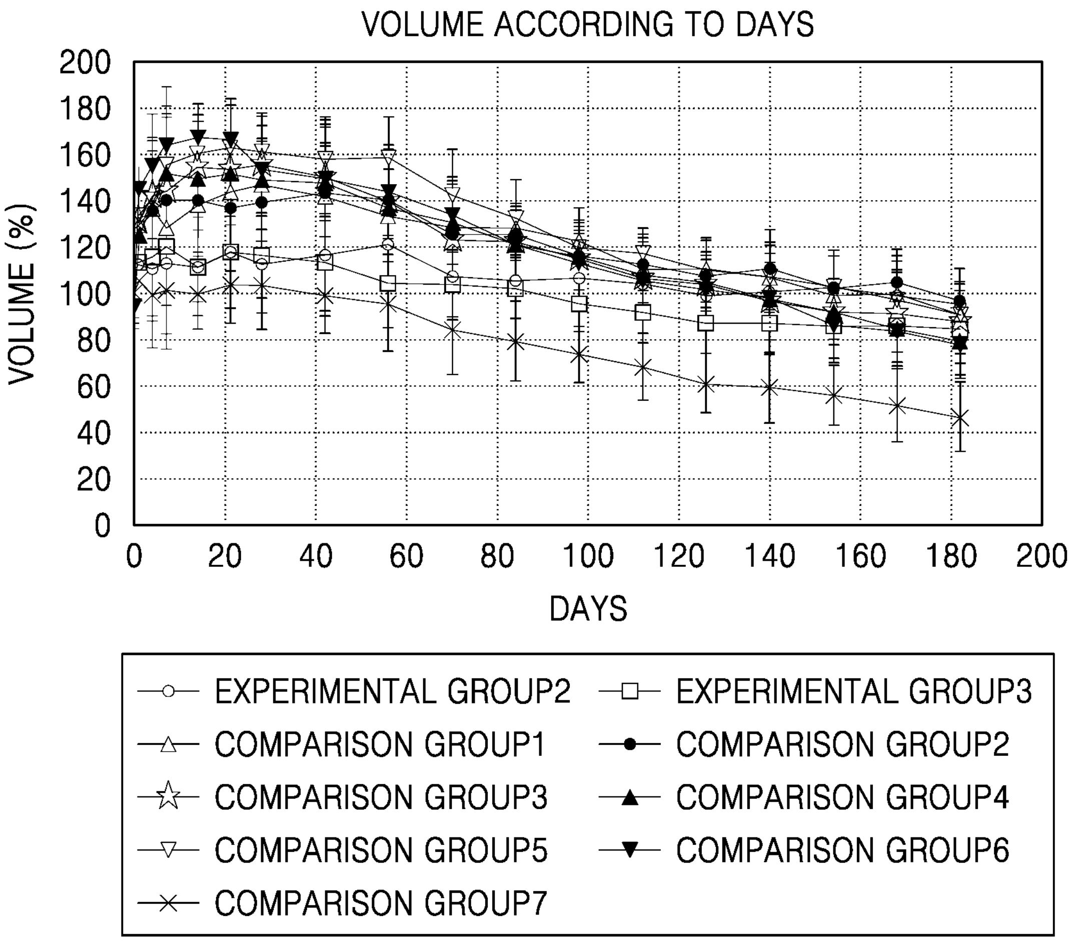
VOLUME ACCORDING TO DAYS
VOLUME (%)
0
20
40
60
80
100
120
140
160
180
200
DAYS
0
20
40
60
80
100
120
140
160
180
200
EXPERIMENTAL GROUP2
EXPERIMENTAL GROUP3
COMPARISON GROUP1
COMPARISON GROUP2
COMPARISON GROUP3
COMPARISON GROUP4
COMPARISON GROUP5
COMPARISON GROUP6
COMPARISON GROUP7

## INTERNATIONAL SEARCH REPORT

International application No.
**PCT/KR2021/001989**

**A. CLASSIFICATION OF SUBJECT MATTER**

**C08B 37/08**(2006.01)i; **C08J 3/24**(2006.01)i; **A61L 27/20**(2006.01)i

According to International Patent Classification (IPC) or to both national classification and IPC

**B. FIELDS SEARCHED**

Minimum documentation searched (classification system followed by classification symbols)

C08B 37/08(2006.01); A61K 31/167(2006.01); A61K 47/48(2006.01); A61K 9/00(2006.01); A61K 9/16(2006.01); A61L 27/20(2006.01); A61L 27/54(2006.01); C08J 3/075(2006.01); C08J 3/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산(hyaluronic acid), 가교(crosslink), 단일상(monophasic), 이성상 (biphasic), 임계 변형률(critical strain), 점착력(adhesive force)

**C. DOCUMENTS CONSIDERED TO BE RELEVANT**

| Category* | Citation of document, with indication, where appropriate, of the relevant passages | Relevant to claim No. |
|---|---|---|
| X | KR 10-1869988 B1 (LG CHEM, LTD.) 21 June 2018 (2018-06-21)<br>See paragraphs [0021] and [0039]-[0050]; examples 1-6; and table 1. | 1-3 |
| A | KR 10-1660211 B1 (DONG KOOK PHARM. CO., LTD.) 26 September 2016 (2016-09-26)<br>See paragraph [0029]; examples 1-4; and tables 1 and 2. | 1-3 |
| A | US 2015-0283080 A1 (MODI, Pankaj) 08 October 2015 (2015-10-08)<br>See claims 1-10. | 1-3 |
| A | US 9782490 B2 (ANTEIS S.A.) 10 October 2017 (2017-10-10)<br>See claim 12; and example 1. | 1-3 |
| A | CN 108250457 A (SHANGHAI LIKANGRUI BIOLOGICAL ENGINEERING CO., LTD.) 06 July 2018 (2018-07-06)<br>See claim 1; and example 1. | 1-3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 May 2021** | **24 May 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/001989**

**C. DOCUMENTS CONSIDERED TO BE RELEVANT**

| Category* | Citation of document, with indication, where appropriate, of the relevant passages | Relevant to claim No. |
|---|---|---|
| A | KR 10-2018-0099550 A (CG BIO CO., LTD. et al.) 05 September 2018 (2018-09-05) See claims 1, 6 and 7; and table 6. | 1-3 |

Form PCT/ISA/210 (second sheet) (July 2019)

**INTERNATIONAL SEARCH REPORT**

International application No.
**PCT/KR2021/001989**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **7, 9**
because they relate to parts of the international application that do not comply with the prescribed requirements to such an extent that no meaningful international search can be carried out, specifically:

Claims 7 and 9 refer to multiple dependent claims not meeting the requirement of PCT Rule 6.4(a).

3. ☑ Claims Nos.: **4-6, 8, 10-13**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/001989**

| Patent document cited in search report | Publication date (day/month/year) | Patent family member(s) | Publication date (day/month/year) |
|---|---|---|---|
| KR 10-1869988 B1 | 21 June 2018 | AR 093689 A1 | 17 June 2015 |
| | | BR 102013030668 A2 | 04 August 2015 |
| | | CN 110078978 A | 02 August 2019 |
| | | IN 3773MU2013 A | 07 August 2015 |
| | | MX 2013013828 A | 26 September 2014 |
| | | RU 2568998 C2 | 20 November 2015 |
| | | TR 201313318 A2 | 21 October 2014 |
| | | TW 201436827 A | 01 October 2014 |
| KR 10-1660211 B1 | 26 September 2016 | WO 2017-213404 A1 | 14 December 2017 |
| US 2015-0283080 A1 | 08 October 2015 | None | |
| US 9782490 B2 | 10 October 2017 | AU 2014-280544 A1 | 03 December 2015 |
| | | BR 112015031026 B1 | 07 July 2020 |
| | | CA 2914417 A1 | 18 December 2014 |
| | | CN 105358186 B | 08 September 2017 |
| | | DK 3007737 T3 | 27 November 2017 |
| | | EP 3007737 A1 | 20 April 2016 |
| | | ES 2647227 T3 | 20 December 2017 |
| | | FR 3006689 A1 | 12 December 2014 |
| | | HK 1221669 A1 | 09 June 2017 |
| | | IL 242657 A | 30 January 2020 |
| | | JP 2016-527338 A | 08 September 2016 |
| | | JP 6491199 B2 | 27 March 2019 |
| | | KR 10-2016-0031465 A | 22 March 2016 |
| | | LT 3007737 T | 11 December 2017 |
| | | MX 2015016835 A | 11 April 2016 |
| | | PL 3007737 T3 | 30 March 2018 |
| | | RU 2015154035 A | 14 July 2017 |
| | | SG 11201510103 A | 28 January 2016 |
| | | US 2016-0144043 A1 | 26 May 2016 |
| | | WO 2014-198406 A1 | 18 December 2014 |
| | | ZA 201508544 B | 30 November 2016 |
| CN 108250457 A | 06 July 2018 | None | |
| KR 10-2018-0099550 A | 05 September 2018 | AU 2018-228301 A1 | 19 September 2019 |
| | | BR 112019017786 A2 | 31 March 2020 |
| | | CN 110573189 A | 13 December 2019 |
| | | EP 3590546 A1 | 08 January 2020 |
| | | JP 2020-508789 A | 26 March 2020 |
| | | TW I707707 B | 21 October 2020 |
| | | US 10980829 B2 | 20 April 2021 |
| | | WO 2018-159984 A1 | 07 September 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017213404 A1


### Non-patent literature cited in the description


- **GANJI, FARIBA ; VASHEGHANI FARAHANI, SAMIRA ; VASHEGHANI-FARAHANI, EBRAHIM.** Theoretical Description of Hydrogel Swelling: A Review. *Iranian Polymer Journal,* 2010, vol. 19, 375-398